(19) Europäisches Patentamt
European Patent Office
Office européen des brevets

(11) EP 3 482 692 A1

(12) EUROPEAN PATENT APPLICATION
published in accordance with Art. 153(4) EPC

(43) Date of publication:
15.05.2019 Bulletin 2019/20

(51) Int Cl.:
*A61B 8/14* (2006.01)

(21) Application number: 16908133.8

(22) Date of filing: 05.07.2016

(86) International application number:
PCT/JP2016/069905

(87) International publication number:
WO 2018/008089 (11.01.2018 Gazette 2018/02)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Hitachi, Ltd.
Tokyo 100-8280 (JP)

(72) Inventors:
• HIROSHIMA, Misaki
  Tokyo 100-8280 (JP)
• TANAKA, Tomohiko
  Tokyo 100-8280 (JP)
• TANAKA, Chizue
  Tokyo 100-8280 (JP)
• IKEDA, Teiichiro
  Tokyo 100-8280 (JP)

(74) Representative: Strehl Schübel-Hopf & Partner
Maximilianstrasse 54
80538 München (DE)

(54) **SPECTRUM ANALYSIS DEVICE, SPECTRUM ANALYSIS METHOD, AND ULTRASONIC IMAGING DEVICE**

(57)    Accuracy of spectrum analysis improves, and further reliability of information indicating tissue characterization of a living organ improves.

A computation region, which is a target of frequency spectrum analysis, and a window region are set on a received signal. A plurality of received signals of the window region are weighted and the frequency spectrum analysis is performed on the received signals of the window region after the weighting. The weighting is performed by using weight distribution corresponding to strength distribution generated in the received signals of the window region in a case of assuming that the ultrasound transmitted from the plurality of transducers propagates as waves in the subject, reaches a target region in the subject which corresponds to the computation region, is reflected from the target region, then further propagates as waves in the subject, and reaches the plurality of transducers.

Fig.6

WINDOW REGION 32

## Description

Technical Field

The present invention relates to a spectrum analysis device that analyzes a frequency spectrum of a received signal obtained, based on a transmitted/received wave of ultrasound to/from a subject and an ultrasonic imaging device that acquires information indicating tissue characterization of the subject, based on results of spectrum analysis by the spectrum analysis device.

Background Art

In the related art, there has been known an ultrasonic diagnostic device that transmits an ultrasonic beam to living tissue, receives and analyzes a reflected wave (echo signal) thereof, and generates a diagnostic image or the like. In recent years, since there has been a demand for an ultrasonic diagnostic device that not only generates the diagnostic image but also diagnoses tissue characterization of a living organ such as a blood vessel or various types of internal organs, there has been proposed an ultrasonic diagnostic device that measures physical quantity (hereinafter, biological physical quantity) in a living organ. Examples of the biological physical quantity include a sonic attenuation rate, a sound speed, or bloodstream of living tissue, moving rate or scattering characteristic quantity of the living tissue, or the like. Hence, in order to measure biological physical quantity, there is used a technology of using a spectral change of a longitudinal wave (ultrasonic carrier) due to the biological physical quantity.

As an example of such an ultrasonic diagnostic device, PTL 1 discloses a living tissue characterization diagnostic device that sets a gate (window region) at each of a plurality of positions of echo signals and diagnoses living tissue characterization by performing fast Fourier transform processing on the echo signals within the gate and obtaining frequency characteristics.

Citation List

Patent Literature

[PTL 1] JP-A-2001-170046

Summary of Invention

Technical Problem

However, a problem arises in that the echo signals within the gate (window region) include not only spectral information of ultrasound emitted from a target region in a living organ which corresponds to a gate position but also spectral information of ultrasound emitted from a peripheral region of the target region. Therefore, an analysis result obtained by performing spectrum analysis on the echo signal of the window region includes information of the peripheral region, in addition to information of the target region in the living organ, and thus the resolution of information indicating tissue characterization of the target region is degraded.

The present invention is made in consideration of the circumstance described above, and an object thereof is to improve the accuracy of spectrum analysis and further to improve the reliability of information indicating tissue characterization of a living organ.

Solution to Problem

In order to solve the problem, the present invention provides the following means.

According to an aspect of the present invention, there is provided a spectrum analysis device including: a region setting unit that sets a computation region which is a target of frequency spectrum analysis of received signals and a window region including the computation region, with respect to the received signals obtained after phasing addition of respective received signals which are each obtained in time series by receiving ultrasound with a plurality of transducers, the ultrasound being transmitted from the plurality of arranged transducers to a subject and being reflected from or penetrating the subject; and a spectrum extracting unit that weights the plurality of received signals of the window region and performs frequency spectrum analysis of the received signals of the window region after the weighting. The spectrum extracting unit performs the weighting by using weight distribution corresponding to strength distribution generated in the received signals of the window region in a case of assuming that the ultrasound transmitted from the plurality of transducers propagates as waves in the subject, reaches a target region in the subject which corresponds to the computation region, is reflected from or penetrates the target region, then further propagates as waves in the subject, and reaches the plurality of transducers.

Advantageous Effects of Invention

According to the present invention, it is possible to improve accuracy of spectrum analysis and further to improve reliability of information indicating tissue characterization of a living organ.

Brief Description of Drawings

Fig. 1 is a perspective view illustrating a schematic configuration of an ultrasonic imaging device according to an embodiment of the present invention.
Fig. 2 is a block diagram illustrating the schematic configuration of the ultrasonic imaging device according to the embodiment of the present invention.
Figs. 3 (a) to 3 (e) are diagrams illustrating a procedure of processing of a received signal based on transmission/reception of ultrasound and a computation region and a window region which are set on the received signal.
Fig. 4 is a block diagram illustrating a configuration of a weight computing unit of the embodiment.
Fig. 5 is a block diagram illustrating a configuration of a spectrum analyzing unit of the embodiment.
Fig. 6 is a diagram illustrating an example of wave weight of the embodiment.
Fig. 7 is a diagram illustrating the computation region and subregions of the embodiment.
Fig. 8 is a diagram illustrating an example of giving a weight to a window region in the related art.
Fig. 9 is a graph obtained by comparing a case where a spectrum energy rate of ultrasound from each position of a target region 30 is shown with respect to total energy of spectra of the embodiment and the window region is weighted according to the related art to a case where weighting of an ultrasonic imaging device according to the embodiment of the present invention is performed.
Fig. 10 is a diagram illustrating an example of a computation pixel of biological physical quantity set on a B-mode image of the embodiment.
Fig. 11 is a diagram illustrating a map of the biological physical quantity of the embodiment.
Fig. 12 is a flowchart illustrating a determination process of weight distribution by using spectrum analysis in the ultrasonic imaging device according to the embodiment.
Fig. 13 is a flowchart illustrating a spectrum analyzing process in the ultrasonic imaging device according to the embodiment.

Description of Embodiments

Hereinafter, an ultrasonic imaging device according to an embodiment of the present invention will be described with reference to the figures.

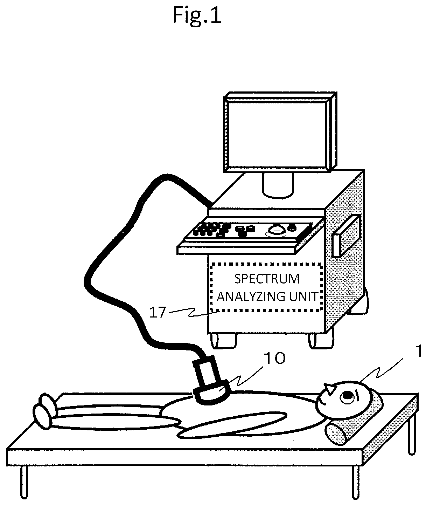

Fig. 1 is a perspective view illustrating a schematic configuration of an ultrasonic imaging device (ultrasound transmitting/receiving device) according to the embodiment. An ultrasonic imaging device 2 includes a spectrum analyzing unit (spectrum analysis device) 17, which performs frequency spectrum analysis of received signals which are each obtained in time series based on a transmitted/received wave of ultrasound to a subject 1, and acquires biological physical quantity indicating tissue characterization by transmitting an ultrasonic pulse and analyzing a received signal obtained from the subject 1, in addition to acquiring and displaying a so-called B-mode image.

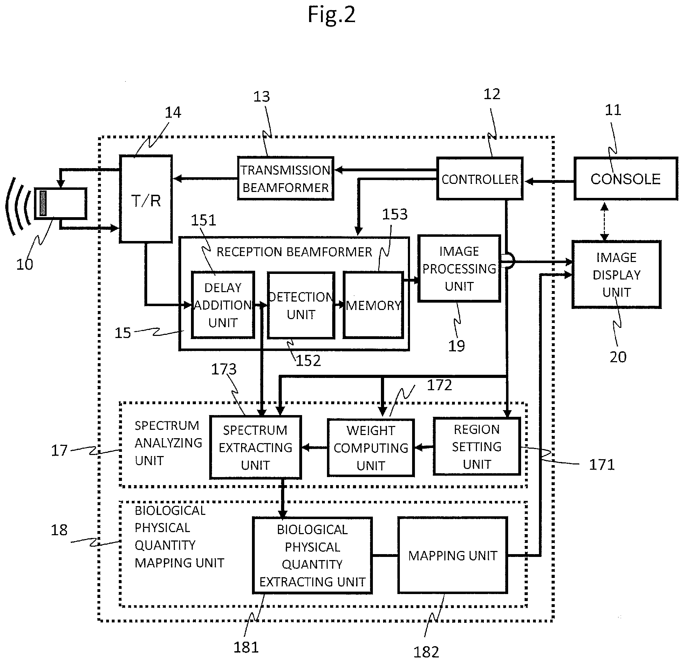

Therefore, as illustrated in Fig. 2, the ultrasonic imaging device includes an ultrasound probe 10, a console 11 that receives an input from an operator, a controller 12, a transmission beamformer 13, a transmit/receive separating circuit (T/R: transmit/receive) 14, a reception beamformer 15, the spectrum analyzing unit 17, a biological physical quantity mapping unit 18, an image processing unit 19, and an image display unit 20.

The console 11 is configured to include a switch group, a keyboard, or the like and receives an input from an operator. The controller 12 controls units and members, in addition to notifying the transmission beamformer 13 of an ultrasound transmission start instruction or the like. In addition, the controller 12 sets, to the transmission beamformer 13, a transmission pulse setting value (the center frequency, a bandwidth, an amplitude of a transmission signal waveform, and duration of a transmission pulse (a frequency of a carrier waveform contained in the transmission pulse)), a coordinate of an opening used for transmission, a setting value of a transmission beam (a transmission focal position), or the like. The transmission beamformer 13 generates a transmission signal based on the transmission pulse setting value and the setting value of the transmission beam which are received from the controller 12, delays the transmission signal for each transducer in the opening as a region of the transducer which contributes to transmission according to the transmission focal position, and outputs a delayed transmission signal to each of the transducers in the transmission opening of the ultrasound probe 10 via the transmit/receive separating circuit 14.

The ultrasound probe 10 includes an electroacoustic transducer array configured of a plurality of arranged transducers. The transducer is configured of a piezoelectric body that converts an electric signal (voltage waveform) into a mechanical stress signal (sound pressure waveform). The transmission opening is a region of transducers that perform irradiation with the ultrasound in a predetermined direction and is configured of one or more transducers. The transducers are each driven based on the transmission signal supplied from the transmit/receive separating circuit 14 and transmit the ultrasound toward the subject 1. One or more transducers in the transmission opening transmit the ultrasound, and thereby the inside of the subject 1 is irradiated with transmission beams as the ultrasonic pulses, which are focused on a transmission focal point. The transmission focal point may be virtually positioned outside the subject 1 (on a front side of the ultrasound probe with respect to a transmission direction of the ultrasound), in addition to a case of being positioned inside the subject 1.

The transmission beam, with which the inside of the subject 1 is irradiated, is reflected from the inside of the subject 1 or penetrates the subject 1 and is received by the transducer of the ultrasound probe 10. In a case where the ultrasound penetrating the subject 1 is received, the ultrasound probe 10 having a circular ring shape or a pair of ultrasound probes 10 disposed to face each other with the subject 1 interposed therebetween is used. In the following description, a case where the ultrasound probe 10 receives the ultrasound reflected from the inside of the subject 1 will be described as an example.

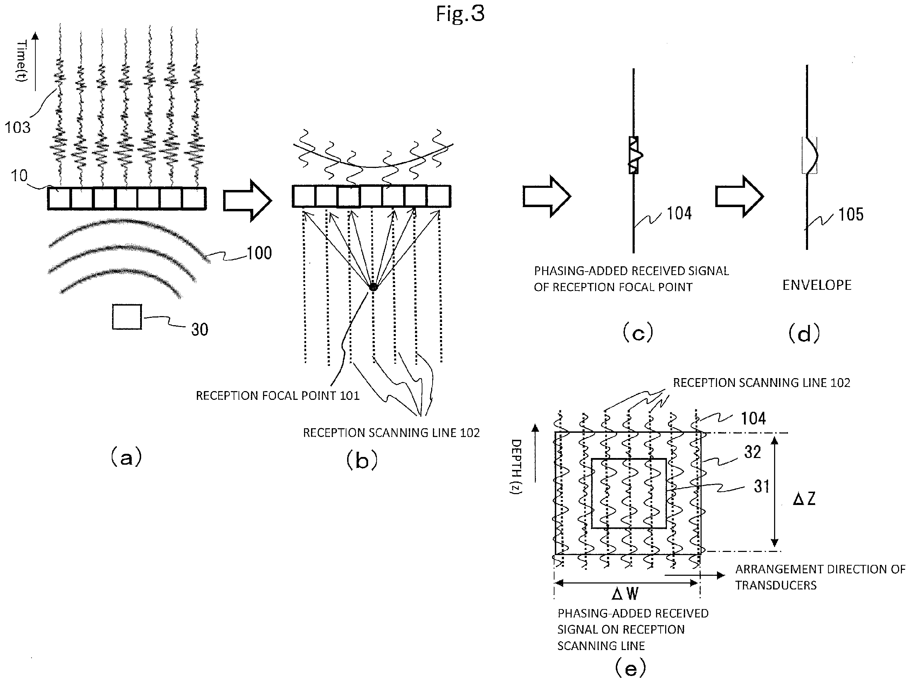

As illustrated in Fig. 3 (a), the transmission beam, with which the inside of the subject 1 is irradiated, is transmitted, is scattered backward, and the like from a reflective body in the subject 1, thereby generating an echo 100 and reaching the ultrasound probe 10. The plurality of transducers of the ultrasound probe 10 convert the sound pressure waveform of the echo into a voltage waveform so as to each generate a plurality of received signals 103, and the generated received signals 103 are output to the reception beamformer 15.

As illustrated in Fig. 2, the reception beamformer 15 includes a delay addition unit 151, a detection unit 152, and a memory 153 and performs A/D conversion of the plurality of received signals by an A/D converting circuit (not illustrated) . Then, the delay addition unit 151 generates phasing-added received signals 104 by adding the received signals 103 after the received signals 103 are each delayed by a delay time depending on a position of a reception focal point 101 set on a predetermined reception scanning line 102, as illustrated in Figs. 3(a), 3(b), and 3(c). As illustrated in Fig. 3(d), the detection unit 152 detects an envelope of the phasing-added received signal 104. The amplitude of the received signal 105 after the detection of the envelope corresponds to the strength of the echo generated on the reception focal point 101.

A plurality of reception scanning lines 102 are set at predetermined intervals within an imaging range, and a plurality of reception focal points 101 are set at predetermined intervals on the reception scanning lines 102. Therefore, the delay addition unit 151 sequentially delays and adds the received signals 103 at the plurality of reception focal points 101 on the respective reception scanning lines 102, and thereby the phasing-added received signal 104 is generated for each reception scanning line 102, as illustrated in Fig. 3(e). The detection unit 152 sequentially detects the envelopes of the phasing-added received signals 104 in time series and detects the echo strength at the plurality of reception focal points 101 on the reception scanning lines 102. Further, the reception beamformer 15 stores the generated phasing-added received signals 104 and the received signals 105 obtained after the detection to the memory 153.

The image processing unit 19 receives, from the memory 153 of the reception beamformer 15, the echo strength for each reception scanning line 102 at each reception focal point 101 after the detection of the envelope and generates an ultrasonic image (for example, a B-mode image). The generated ultrasonic image is displayed on the image display unit 20. For example, a process for generating the ultrasonic image by the image processing unit 19 includes a logarithmic compression process or the like.

Here, the waveform of the received signal 103 that is received by the reception beamformer 15 and the phasing-added received signal 104 that is generated by the reception beamformer includes information of a carrier waveform which is a waveform obtained by superimposing the transmission signal waveforms of the ultrasound through frequency attenuation or phase rotation of the waveforms in a propagation procedure, in addition to amplitude information (strength information) for generating the B-mode image. The information of the carrier waveform includes information of a change in waveform such as a frequency change or phase rotation which is generated in the propagation procedure of the ultrasound and time information obtained when the ultrasound propagates in the subject. The information of the carrier waveform indicates information of a propagation medium (subject 1). In the embodiment, the spectrum analyzing unit 17 performs the spectrum analysis of the received signal obtained from the reception beamformer 15 and further extracts the information of the subject 1.

At this time, when viewed from any reflection point in the subject 1, the transmission and the reception of the ultrasound are each performed in accordance with directionality (transmission directionality and reception directionality). Therefore, from a convolution operation of the transmission/reception of the ultrasound and the reflection of a scattering body, the ultrasound obtained when the ultrasound reaches the ultrasound probe 10 has a predetermined spread represented by a point-spread function. Therefore, the received signal detected by the ultrasound probe 10 is reflected from a plurality of reflection points of the subject 1 and becomes a signal obtained by superimposing waves of the ultrasound which are each spread by the point-spread function. In other words, the received signal output from the transducer includes superimposed spectrum information emitted from various peripheral points, in addition to the spectrum information at a predetermined reflection point.

In the embodiment, as illustrated in Fig. 3(e), a window region 32 is set to surround a computation region 31 of the received signal, which corresponds to a predetermined target region 30 of the subject 1. The received signals in the window region 32 are weighted, and thereby signals from the peripheral of the target region 30, which are included in the received signals, are removed (attenuated). In this manner, it is possible to extract the spectrum information in the target region 30 with high accuracy. Hereinafter, this will be described further in detail.

The spectrum analyzing unit 17 includes a region setting unit 171, a weight computing unit 172, and a spectrum extracting unit 173.

The region setting unit 171 sets the computation region 31 which is a target of frequency spectrum analysis and the window region 32 which includes the computation region, on the received signals which are each obtained from the transducers in time series . Here, the received signal as a target, on which the computation region 31 and the window region 32 are set, is the phasing-added received signal 104 generated in the delay addition unit 151 of the reception beamformer 15, as illustrated in Fig. 3(e).

As illustrated in Fig. 3(e), the region setting unit 171 sets the computation region 31 on which the spectrum analysis is performed and the window region 32 set to surround the computation region 31, on a plurality of phasing-added received signalsl04. In other words, the region setting unit 171 sets the computation region 31 and the window region 32 on the plurality of phasing-added received signals 104 disposed in a two-dimensional space in which a depth direction (a distance from a probe front surface) and an arrangement direction of the plurality of transducers are axes of the space. The region setting unit 171 performs and determines computation by a predetermined computation method based on a propagation speed of the ultrasound and a position and a size of the target region 30 such that the position and size of the computation region 31 correspond to a range of the received signal generated when the transmission beam is reflected from the target region 30, of which the information indicating the tissue characterization of the living organ in the subject 1 is wanted to be obtained and generated echo 100 reaches the transducer. The position and the size of the target region 30 are received from a user via the console 11 and the controller 12.

In addition, the positions and the sizes of the computation region 31 corresponding to the target region 30 having a plurality of types of positions and sizes which are settable by a user, may be obtained from the computation in advance, or the computation result may be stored in the memory 41 in the region setting unit 171. In this case, the region setting unit 171 reads, from the memory 41, the position and the size of the computation region 31 corresponding to the target region 30, which are received from the user via the console 11 and the controller 12, and sets the computation region on the phasing-added received signals 104.

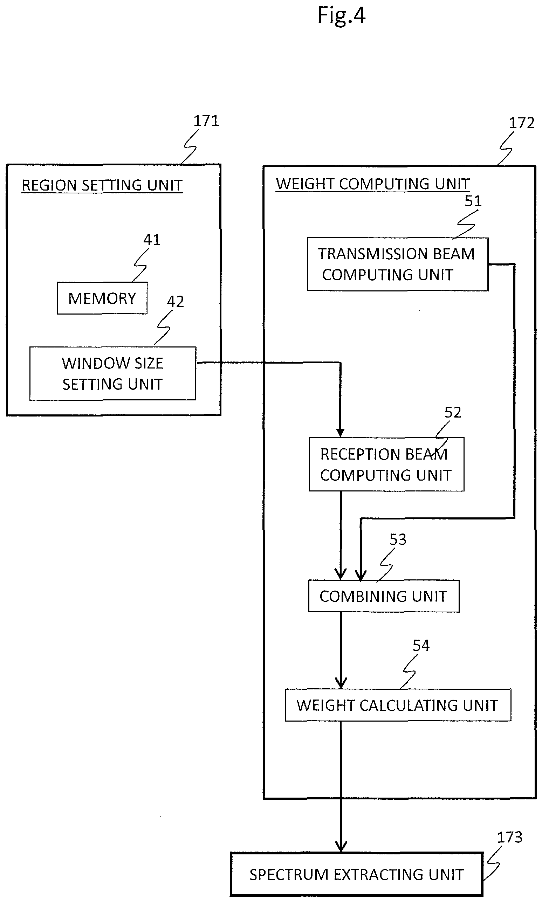

The region setting unit 171 includes a window size setting unit 42 that has a configuration illustrated in Fig. 4. The window size setting unit 42 gives, to a reception beam computing unit 52, a size $\Delta Z$ of the window region 32 in the depth direction, which is positioned on an outer side from the computation region 31 and a size $\Delta W$ (refer to Fig. 3(e)) of the transducer in the arrangement direction. The window size setting unit 42 obtains the size $\Delta Z$ of the window region 32 in the depth direction from computation based on the predetermined frequency resolution and a sound speed c of the ultrasound in the subject 1 such that predetermined frequency resolution is obtained in the computation region 31. For example, the window size setting unit 42 calculates the size in accordance with Equation (1) representing a relationship between the length $\Delta Z$ and the frequency resolution of the space window which is derived from a relationship between the length of the time window and the predetermined frequency resolution in the Fourier transform.
Equation (1)

$$\Delta Z = \frac{1}{2} \cdot \frac{c}{\Delta F} \qquad \ldots (1)$$

Here, c represents a sound speed value, and $\Delta F$ represents the predetermined frequency resolution.

On the other hand, the window size setting unit 42 obtains $\Delta W$ as the size of the window region 32 positioned on the outer side from the computation region 31 in the arrangement direction of the transducers such that the predetermined frequency resolution is obtained similarly to $\Delta Z$. For example, since both of $\Delta W$ and $\Delta Z$ are the space directions, the same relationship as Equation (1) is also established for $\Delta W$, $\Delta W$ is calculated in accordance with Equation (2) representing a relationship between $\Delta W$ and the frequency resolution.

$$\Delta W = \frac{1}{2} \cdot \frac{c}{\Delta F} \qquad \dots(2)$$

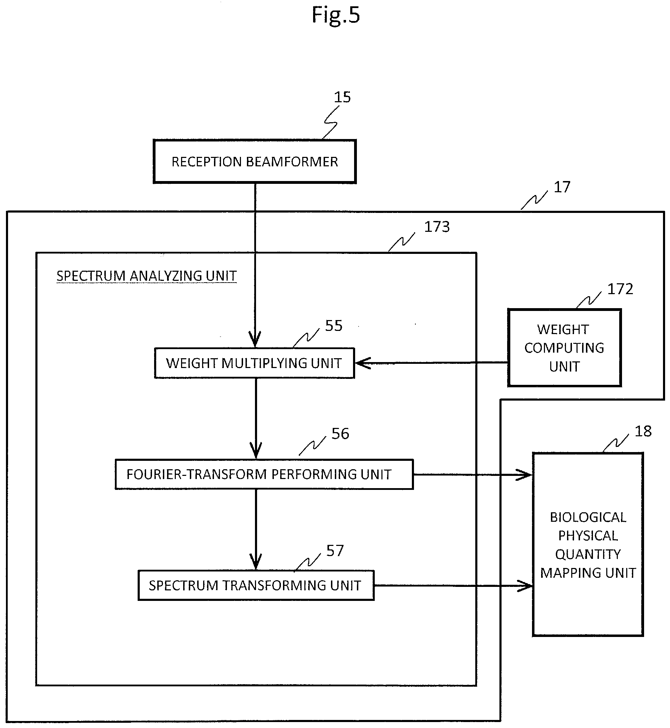

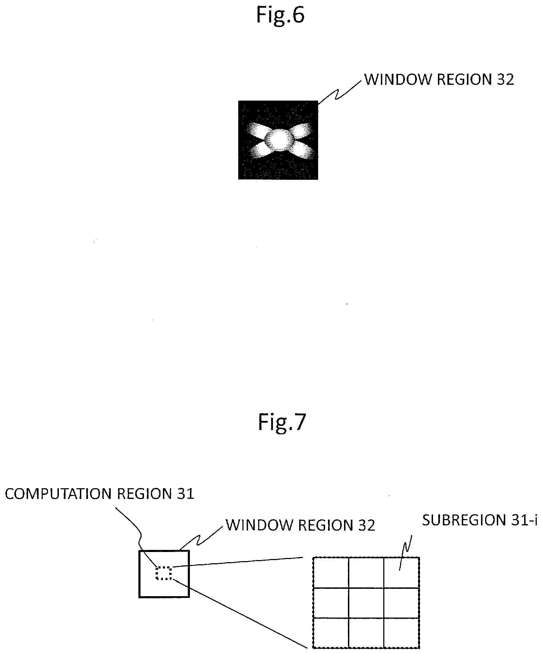

As illustrated in Fig. 5, the spectrum extracting unit 173 includes a weight multiplying unit 55, a Fourier-transform performing unit 56, and a spectrum transforming unit 57. The weight multiplying unit 55 gives a weight to the plurality of phasing-added received signals 104 including the window region 32. The Fourier-transform performing unit 56 and the spectrum transforming unit 57 perform the frequency spectrum analysis of the phasing-added received signals 104 of the window region 31 after the weighting and transmit a waveform spectrum and a frequency spectrum which are results thereof to the biological physical quantity mapping unit 18. At this time, the spectrum extracting unit 55 performs the weighting by using weight distribution (for example, refer to Fig. 6) corresponding to strength distribution generated in the phasing-added received signals 104 of the window region 32 in a case of assuming that the ultrasound (transmission beam) transmitted from the plurality of transducers propagates as waves in the subject 1, reaches the target region 30 in the subject 1 which corresponds to the computation region 31, is reflected from the region, then further propagates as waves in the subject 1, and reaches the plurality of transducers. In the embodiment, the weight distribution determined in consideration of such wave propagation is referred to as "wave weight".

As described above, the phasing-added received signal 104 in the window region 32 is weighted by using the wave weight, and thereby it is possible to remove (attenuate) the received signal of the echo generated in the peripheral region of the target region 30, which is included in the phasing-added received signal 104. Accordingly, in the spectrum analysis after the weighting, it is possible to obtain a spectral change generated in the ultrasound (transmission beam) through propagation in the target region 30 from the transducer, a spectral change generated due to the reflection or the scattering in the target region 30, and a spectral change generated in the ultrasound (echo) in a path through which propagation is performed from the target region 30 to the transducer, with high accuracy.

The weight computing unit 172 obtains the wave weight by computation that is used for weighting the weight multiplying unit 55 of the spectrum extracting unit 173. As illustrated in Fig. 4, the weight computing unit 172 includes a transmission beam computing unit 51, the reception beam computing unit 52, a combining unit 53, and a weight calculating unit 54.

The transmission beam computing unit 51 calculates strength distribution of the ultrasound in the target region 30 in a case of assuming that the ultrasound transmitted from the plurality of transducers propagates in the subject 1 and reaches the target region 30.

The reception beam computing unit 52 calculates strength distribution of the phasing-added received signal 104 in the window region 32 in a case of assuming that the ultrasound emitted from the target region 30 irradiated with the ultrasound having predetermined strength distribution propagates in the subject, reaches the transducer, and is received by the transducer.

The combining unit 53 obtains strength distribution generated in the phasing-added received signals 104 of the window region 32 by combining the strength distribution calculated by the transmission beam computing unit 51 with the strength distribution calculated by the reception beam computing unit 52. The weight calculating unit 54 determines the weight distribution based on the strength distribution generated in the phasing-added received signal 104 of the window region 32, which is obtained by the combining unit 53. Hereinafter, this is specifically described using equations.

For example, the transmission beam computing unit 51 computes, from Equation (3), the strength of the ultrasound in an imaging region, that is, a beam sound field $A_{tx}$ (x, z) at a position (x, z) in the target region 30, in a case of assuming that the ultrasound transmitted from the transducer reaches the imaging region including the target region 30.

$$A_{tx}(x, y) = \sum_{M} w_{tx}(m)/r \int P(f) \exp\{j2\pi f(t - r/c - \tau(m))\} df \qquad \dots(3)$$

Here, m represents number of the transmission opening, M represents the number of transducers of the transmission opening, $W_{tx}$ represents an opening weight applied to the transmission opening, P(f) represents a function representing a spectrum of a frequency f of the transmission waveform, c represents a sound speed value, r represents a distance between the transducer of the transmission opening and a point (x, z) in the target region 30, which is used in the transmission given in the following Equation (4), $\tau$ represents a transmission delay time of the transmission opening, and t represents time.

$$r = \sqrt{\left(x - x_e(m)\right)^2 + \left(z - z_e(m)\right)^2} \qquad \ldots(4)$$

Here, $x_e$ (m) and $z_e$ (m) are an x coordinate and a z coordinate of an m-th transmission element.

The reception beam computing unit 52 calculates strength distribution of the phasing-added received signal 104 in the window region 32, which is output from the plurality of transducers, that is, a reception beam sound field $A_{rx}$ (x, z) at the position (x, z) in the window region 32, from the following Equation (5), in a case of assuming that the ultrasound emitted from the target region 30 irradiated with the ultrasound having the predetermined strength distribution propagates in the subject 1, reaches the transducer, and is received by the transducer.

$$A_{rx}(x,z) = \sum_M w_{rx}(m)/r \int P'(f) \exp\{j2\pi f(t - r/c)\} df \qquad \ldots(5)$$

Here, m represents number of the reception opening, M represents the number of transducers of the transmission opening, P'(f) represents a function representing a spectrum of a frequency f of the transmission waveform, and, for example, this is calculated and set from the spectrum P(f) of the transmission waveform and an impulse response of the reception. $W_{rx}$ represents an opening weight applied to the reception opening, r represents a distance between the reception transducer and the point (x, z) in the target region 30, which is calculated similarly to Equation (3), c represents a sound speed value, and t represents time.

The reception beam computing unit 52 divides the computation region 31 into a plurality of (one) subregions 31-i (i = 1, 2, ... I) as illustrated in Fig. 7, when calculating the reception beam sound field $A_{rx}$ (x, z), and computes a reception beam sound field $A_{rx}$ (x(i), y(i)) for each subregion 31-i by using Equation (5). For example, the size of the subregion 31-i is set to a size to about a subwavelength. The subwavelength is equal to or smaller than the strength of the reception pulse.

Hence, the combining unit 53 performs combination by obtaining a product of the transmission beam sound field $A_{tx}$ (x, z) calculated by the transmission beam computing unit 51 and the reception beam sound field $A_{rx}$ (x(i), z(i)) for each subregion 31-i, which is calculated by the reception beam computing unit 52, and obtains signal strength distribution $A_w$ generated on the received signal 104 of the window region 32. Here, the calculation is performed in accordance with the following Equation (6).

$$A_w = A_{tx} \times \sum_I A_{rx}(x(i), z(i)) \qquad \ldots(6)$$

Here, i represents the number of subregions.

The weight calculating unit 54 determines the weight with respect to the window region 32 based on the strength distribution $A_w$ of the signal in the window region 32. For example, the weight is determined in accordance with the following Equation (7). In other words, at a position at which the value of ($A_{wdB}$ + G) obtained by adding the lower limit decibel value G that is a predetermined constant to the decibel value $A_{wdB}$ of the signal strength distribution $A_w$ is equal to or larger than 0, a value $\alpha$ ($A_{wdB}$ + G) obtained by multiplying a predetermined coefficient $\alpha$ to ($A_{wdB}$ +G) is set as a weight $W_w$, and the weight $W_w$ = 0, at a position at which the value of ($A_{wdB}$ + G) is smaller than 0.

Next, the weight calculating unit 54 sets the weight W as 0 with respect to regions other than the region of the window region 32 determined by the region setting unit 171, sets W = $W_w$ in the region of the window region 32, and gives the weight W to the spectrum extracting unit 173. According to the setting, the weight multiplying unit 55 (Fig. 5) multiplies the received signal 104 by W obtained after phasing addition as will be described below, thereby only the received signals 104 after the phasing addition in the region of the window region 32 are extracted, and the wave weight $W_w$ is applied to the received signal 104 in the region of the window region 32.

$$W_w = \alpha(A_{wdB} + G)(A_{wdB} + G \geq 0) \qquad \ldots(7)$$

$$W_w = 0(A_{wdB} + G < 0)$$

Here, $A_{wdB}$ represents a decibel value of the strength distribution $A_w$, G represents the predetermined lower limit decibel value, and $\alpha$ represents a coefficient of a predetermined proportional constant.

The weight $W_w$ is set to the window region 32 as illustrated in Fig. 7, and thereby a weight is applied depending on the signal strength to a point at which the signal strength is larger than the predetermined lower limit value G, and a weight of zero is applied to a point at which the signal strength is smaller than the lower limit value G, based on the signal strength of the window region 32, which is obtained in consideration of the wave propagation from the ultrasound probe 10 (transducer) to the target region 30 and the reflection or the like in the target region 30 and the wave propagation from the target region 30 to the ultrasound probe 10 . In other words, the received signal 104 in the window region 32 is weighted by using the wave weight as illustrated in Fig. 6, which is obtained from equation (7), and thereby it is possible to remove or to attenuate the received signal propagating from a peripheral region of the target region 30, which is included in the received signal 104 in the window region 32.

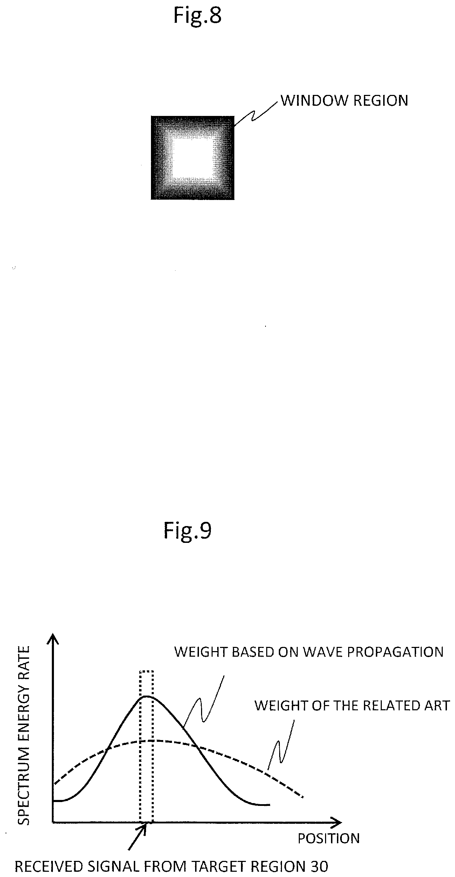

Therefore, in general, as illustrated in Fig. 8, compared to a case where the weight at the center portion used in the spectrum analysis with respect to the window region 32 is the largest and the weight (for example, Hann weight or the like) that is reduced as the position approaches a peripheral portion, the wave weight of the embodiment is used, and thereby it is possible to extract the received signal propagating from the target region 30 in the subject 1 as illustrated in Fig. 9. Then, it is possible to extract the spectrum of the received signal propagating from the target region 30 by spectrum extraction, with high accuracy.

Fig. 9 is a graph obtained by comparing a case where a spectrum energy rate of the ultrasound from each position in the window region 32 is shown with respect to total energy of spectra of the embodiment and the window region 32 is weighted according to the related art to a case where weighting is performed, based on the wave propagation. In Fig. 9, a component of the signal that is scattered and received in the target region 30 is defined as a signal, and a signal that is scattered and received in a region out of the target region 30 is defined as noise. Then, in a case where the weighting is performed, based on the wave propagation, the weighting is performed such that a region, in which the received signal strength from the target region 30, that is, the strength of the signal is larger, remains and a region, in which the signal strength is weak, that is, the noise component is larger, is removed, compared to a case of performing the weight of the related art, the signal to noise ratio (SN ratio) improves, that is, an effect of improving accuracy of the spectrum extraction is obtained.

The weight computing unit 172 may calculate weight distribution from computation whenever a condition of the transmission and reception of the ultrasound is set. In addition, the weight computing unit 172 may be configured to include a weight storing unit that stores the weight distribution obtained from computation in advance for each predetermined settable condition, which is a condition of transmission and reception of ultrasound. In a case where the weight computing unit 172 includes the weight storing unit, the spectrum extracting unit 173 reads, from the weight storing unit, the weight distribution corresponding to the condition of the transmission and the reception of the ultrasound and weights the received signal of the window region 32.

The Fourier-transform performing unit 56 of the spectrum extracting unit 173 performs the frequency spectrum analysis of the phasing-added received signal 104 of the window region 31 weighted by the weight multiplying unit 55 by using the wave weight. In other words, the Fourier-transform performing unit 56 obtains a wavenumber spectrum by performing Fourier transform such as two-dimensional to three-dimensional fast Fourier transform (FFT) on the phasing-added received signal 104 of the window region 32.

In the spectrum transforming unit 57, for example, interpolation processing using an expression of a scattering relationship which is a relationship expression between the frequency and the wavenumber is performed on the wavenumber spectrum data, and thereby the wavenumber spectrum data is converted into a frequency spectrum. The Fourier-transform performing unit 56 and the spectrum transforming unit 57 of the spectrum extracting unit 173 output the wavenumber spectrum and the frequency spectrum as the spectrum information to the biological physical quantity mapping unit 18.

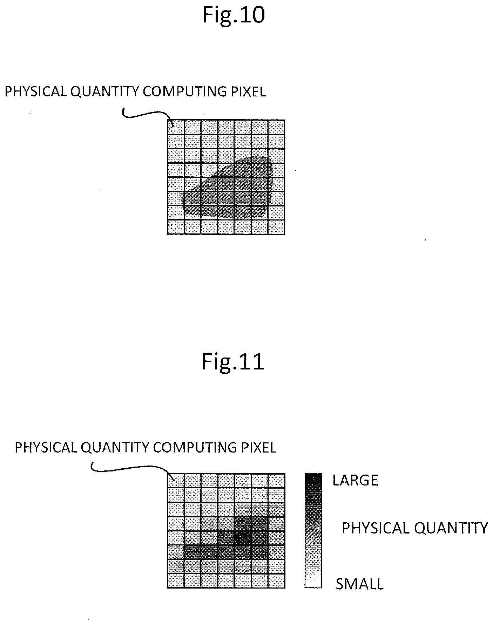

Fig. 10 illustrates an example of a computation pixel of the biological physical quantity, and Fig. 11 illustrates an example of a biological physical quantity map. As illustrated in Fig. 2, the biological physical quantity mapping unit 18 includes a biological physical quantity extracting unit 181 and a mapping unit 182. The biological physical quantity extracting unit 181 stores the wavenumber spectrum and the frequency spectrum acquired from the spectrum extracting unit 173 for each computation pixel of the predetermined biological physical quantity and for every transmission of the transmission beam. The computation pixel is set with the desired resolution as illustrated in Fig. 10 in a part or the entirety of the ultrasonic image (B-mode image) used in diagnosis.

The biological physical quantity extracting unit 181 calculates, by a known computation method, a desired biological physical quantity of the biological physical quantities such as a moving rate, scattering characteristic quantity, or the like of a living organ such as a sonic attenuation rate, a sound speed, or bloodstream of living tissue, from the spectrum information such as the wavenumber spectrum and the frequency spectrum for each computation pixel. For example, the attenuation rate can be calculated by obtaining a difference in amplitude or strength of the frequency components in the depth direction by using the frequency spectrum stored for each computation pixel. The difference may be obtained every time of a plurality of times of transmission, may calculate an average value of the difference between transmission performed several times, and may calculate an attenuation rate based on the average value of the difference. In addition, the moving speed of the bloodstream is computed, based on the change in phase components of the frequency spectrum.

The mapping unit 182 is disposed for each computation pixel by assigning a shade or a hue corresponding to a magnitude of the calculated biological physical quantity in the biological physical quantity extracting unit 181, and thereby a biological physical quantity map is generated as illustrated in Fig. 11. In a case of a bloodstream spectrum, an arrow representing a spectrum may be mapped. The biological physical quantity map may be individually displayed on the image display unit 20 or may be superimposed on the ultrasonic image (B-mode image) generated by the image processing unit 19 and may be displayed on the image display unit 20.

Functions of units included in the spectrum analyzing unit 17 and the biological physical quantity mapping unit 18 are realized as software by reading and executing a program stored in a memory such as a read only memory (ROM) in advance, by a central processing unit (CPU) (not illustrated) included in the controller 12. In addition, some or all of the functions of the units of the spectrum analyzing unit 17 and the biological physical quantity mapping unit 18 may be realized by hardware such as a custom IC of an application specific integrated circuit (ASIC) or a programmable IC of a field-programmable gate array (FPGA).

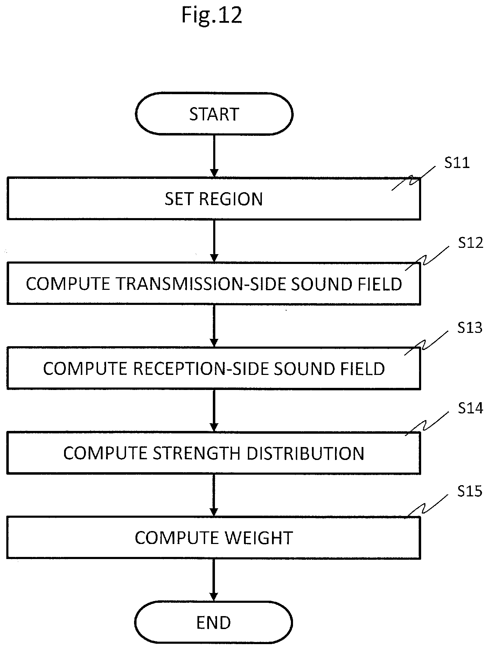

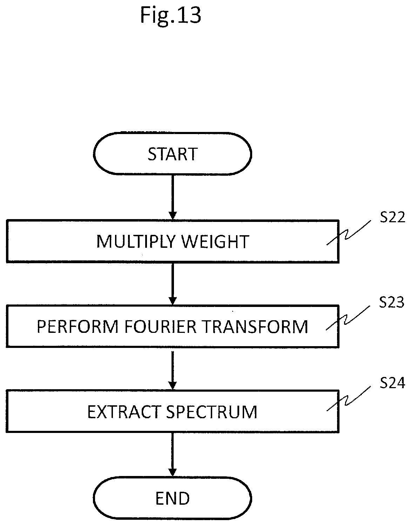

Hereinafter, spectrum analysis processing on the received signal, which is performed by the spectrum analyzing unit 17 in the ultrasonic imaging device according to the embodiment will be described with reference to a flowchart in Figs. 12 and 13.

First, determination processing of weight using the spectrum analysis is described with reference to Fig. 12.

In step S11, the region setting unit 171 sets the computation region which is the target region in which the spectrum analysis is performed and the window region set to surround the computation region. Specifically, the region setting unit 171 receives, from the controller 12, the information related to the position and the size of the target region 30 set by a user in the subject 1, and calculates the position and the size of the computation region 31 on the phasing-added received signal 104 by a predetermined computing method in consideration of the sound rate. Otherwise, the region setting unit 171 reads the position and the size of the computation region 31 which are obtained in advance from the built-in memory 41 based on the information of the position and the size of the target region 30. Further, the window size setting unit 42 of the region setting unit 171 receives the desired frequency resolution from the controller 12 when the spectrum analysis is performed, and calculates the size and the position of the window region 32 based on Equation (1).

In Step S12, the transmission beam computing unit 51 of the weight computing unit 172 computes and calculates the transmission beam sound field $A_{tx}$ (transmission-side sound field) which is transmission strength distribution. Specifically, the weight computing unit 172 receives, from the controller 12, information such as the transmission pulse setting value (the center frequency, the bandwidth, or the like), a transmission opening coordinate, or a transmission beam setting value (focus point), and calculates the transmission beam sound field $A_{tx}$ from the Equation (2) described above.

Next, in Step S13, the reception beam computing unit 52 of the weight computing unit 172 computes and calculates the reception beam sound field $A_{rx}$ (reception-side sound field) which is the reception strength distribution. Specifically, the weight computing unit 172 receives, from the controller 12, the reception pulse setting value, the reception opening coordinate, or the reception beam setting value (reception focus point or the like), and computes and calculates the reception beam sound field $A_{rx}$ in the computation region 31 from the Equation (5). Next, in Step S14, the combining unit 53 of the weight computing unit 172 computes and acquires the strength distribution $A_w$ of the phasing-added received signal 104 in the computation region 31 from Equation (6) by using the transmission beam sound field $A_{tx}$ and the reception beam sound field $A_{rx}$ which are calculated in advance.

The weight calculating unit 54 of the weight computing unit 172 computes and determines the weight $W_w$ with respect to the window region based on the strength distribution $A_w$ in Step S15. Specifically, the weight calculating unit 54 determines the weight distribution (wave weight) with respect to the received signal of the window region 32 from the Equation (7) by using the strength distribution $A_w$ of the received signal in the computation region 32. The weight calculating unit 54 determines the weight $W_w$ with respect to the entire region by using the weight $W_w$ in the window region 32. Specifically, the weight calculating unit 54 determines that $W = 0$ outside the window region 32 and $W = W_w$ in the window region 32.

Subsequently, with reference to Fig. 13, the spectrum analysis processing using the wave weight calculated with reference to the flowchart in Fig. 12 will be described.

In Step S22, the weight multiplying unit 55 of the spectrum extracting unit 173 weights the entirety of the phasing-added received signal 104 by multiplying the wave weight calculated by the weight computing unit 172.

In Step S23, the Fourier-transform performing unit 56 of the spectrum extracting unit 172 calculates the wavenumber spectrum by performing the Fourier transform of the weighted phasing-added received signal 104. In Step S24, the spectrum transforming unit 57 transforms the wavenumber spectrum into the frequency spectrum and extracts the frequency spectrum.

According to the ultrasonic imaging device of the embodiment, since the received signal is weighted in consideration of the spread and strength distribution of the ultrasonic signal which are generated due to the propagation of the ultrasonic signal reaching the target region 30 from the ultrasound probe 10 and propagation of the ultrasonic signal reaching the ultrasound probe 10 from the target region 30, it is possible to extract the spectrum of the target region 30 with high accuracy. Hence, by using the wavenumber or frequency spectrum obtained from such spectrum analysis, it is possible to improve the reliability of the information indicating the tissue characterization of the living organ. Reference Signs List

| | |
|---|---|
| 10: | ultrasound probe |
| 11: | console |
| 12: | controller |
| 13: | transmission beamformer |
| 14: | transmit/receive separating circuit |
| 15: | reception beamformer |
| 17: | spectrum analyzing unit |
| 18: | biological physical quantity mapping unit |
| 19: | image processing unit |
| 20: | image display unit |
| 151: | delay adding unit |
| 152: | detection unit |
| 153: | memory |
| 171: | region setting unit |
| 172: | weight determining unit |
| 173: | spectrum extracting unit |
| 181: | biological physical quantity extracting unit |
| 182: | mapping unit |

## Claims

1. A spectrum analysis device comprising:

   a region setting unit that sets a computation region which is a target of frequency spectrum analysis of received signals and a window region including the computation region, with respect to the received signals obtained after phasing addition of received signals which are each obtained in time series by receiving ultrasound with a plurality of transducers, the ultrasound being transmitted from the plurality of arranged transducers to a subject and being reflected from or penetrating the subject; and

   a spectrum extracting unit that weights the plurality of received signals of the window region and performs frequency spectrum analysis of the received signals of the window region after the weighting,

   wherein the spectrum extracting unit performs the weighting by using weight distribution corresponding to strength distribution generated in the received signals of the window region in a case of assuming that the ultrasound transmitted from the plurality of transducers propagates as waves in the subject, reaches a target region in the subject which corresponds to the computation region, is reflected from or penetrates the target region, then further propagates as waves in the subject, and reaches the plurality of transducers.

2. The spectrum analysis device according to Claim 1, further comprising:

   a weight computing unit that obtains the weight distribution from computation,
   wherein the weight computing unit includes

      a transmission beam computing unit that calculates strength distribution of ultrasound in the target region in a case of assuming that the ultrasound transmitted from the plurality of transducers propagates in the subject and reaches the target region,

a reception beam computing unit that calculates strength distribution of received signals output from the plurality of transducers, which is obtained in the window region, in a case of assuming that the ultrasound emitted from the target region subjected to irradiation of ultrasound having predetermined strength distribution propagates in the subject and reaches the transducers,

a combining unit that obtains strength distribution generated in the received signals of the window region by combining the strength distribution calculated by the transmission beam computing unit with the strength distribution calculated by the reception beam computing unit, and

a weight calculating unit that obtains the weight distribution based on the strength distribution generated in the received signal of the window region.

3. The spectrum analysis device according to Claim 1, further comprising:

a reception beamformer provided with a delay addition unit that generates phasing-added received signals in time series by performing addition after the received signals from the plurality of transducers in time series are each delayed by a delay time corresponding to positions of a plurality of reception focal points on a predetermined reception scanning line and a detection unit that detects an envelope of the phasing-added received signal, wherein the region setting unit sets the window region with respect to the phasing-added received signal generated before the detection performed by the detection unit, and

wherein the spectrum extracting unit weights the phasing-added received signal.

4. The spectrum analysis device according to Claim 2, wherein the reception beam computing unit divides the target region into a plurality of subregions and calculates strength distribution of the received signals of the window region for each subregion, and the combining unit obtains strength distribution generated in the received signals of the window region by adding strength distribution of the received signals of the subregions and combining the added strength distribution of the received signals with the strength distribution calculated by the transmission beam computing unit.

5. The spectrum analysis device according to Claim 2, wherein the weight computing unit further includes a window size setting unit that sets a size of the window region depending on frequency resolution and the size of the computation region which are desirable to results of the frequency spectrum analysis performed by the spectrum extracting unit.

6. The spectrum analysis device according to Claim 1, wherein the spectrum extracting unit performs spectrum analysis from Fourier transform of the weighted received signals of the window region.

7. The spectrum analysis device according to Claim 1, wherein ultrasound that is transmitted from the transducer to the subject has the predetermined center frequency and a predetermined bandwidth.

8. The spectrum analysis device according to Claim 1, wherein the computation region and the window region are set on the plurality of received signals disposed in a space in which a time axis direction of the received signal and an arrangement direction of the plurality of transducers are set as axes, and wherein the spectrum extracting unit performs two-dimensional or three-dimensional Fourier transform of the received signals of the window region.

9. The spectrum analysis device according to Claim 2, further comprising:

a weight storing unit that stores the weight distribution obtained in advance for each predetermined settable condition, which is a condition of transmission and reception of ultrasound of the transducer, and wherein the spectrum extracting unit reads, from the weight storing unit, the weight distribution corresponding to the condition of the transmission and reception of the ultrasound, which is transmitted to the subject and is received by the transducers, and weights the received signals of the window region.

10. The spectrum analysis device according to Claim 1, further comprising:

a reception unit that receives setting of the target region from a user,

wherein the region setting unit includes a computation region calculating unit that obtains the computation region on the received signal from computation, which corresponds to the target region received by the reception unit.

11. A spectrum analysis method comprising:

a region setting step of setting a computation region which is a target of frequency spectrum analysis of received signals and a window region including the computation region, with respect to the received signals which are each obtained in time series by receiving ultrasound with a plurality of transducers, the ultrasound being emitted from the plurality of arranged transducers to a subject and being reflected from the subject; and

a spectrum extracting step of weighting the plurality of received signals of the window region and performing frequency spectrum analysis of the received signals of the window region after the weighting,

wherein, in the spectrum extracting step, weighting is performed by using weight distribution corresponding to strength distribution generated in the received signals of the window region in a case of assuming that the ultrasonic transmitted from the plurality of transducers propagates as waves in the subject, reaches a target region in the subject which corresponds to the computation region, is reflected from the target region, then further propagates as waves in the subject, and reaches the plurality of transducers.

12. An ultrasonic imaging device comprising:

a plurality of arranged transducers;

a transmission beamformer that transmits ultrasound from the transducers to a subject;

a spectrum analysis device that performs spectrum analysis of received signals obtained by receiving, with the plurality of transducers, ultrasound reflected from the subject; and

a biological information extracting unit that calculates biological information of the subject based on analysis results obtained by the spectrum analysis device,

wherein the spectrum analysis device is the spectrum analysis device according to any one of Claims 1 to 10.

Fig.1

SPECTRUM
ANALYZING UNIT

17

10

1

# Fig.2

Fig.3

Time(t)

103

10

100

30

RECEPTION FOCAL POINT 101

RECEPTION SCANNING LINE 102

(a)

(b)

104

PHASING-ADDED RECEIVED SIGNAL
OF RECEPTION FOCAL POINT

(c)

105

ENVELOPE

(d)

RECEPTION SCANNING LINE 102

104

32

31

$\Delta Z$

DEPTH (z)

$\Delta W$

ARRANGEMENT DIRECTION OF
TRANSDUCERS

PHASING-ADDED RECEIVED
SIGNAL ON RECEPTION
SCANNING LINE

(e)

EP 3 482 692 A1

# Fig.4

```
                171                                              172

┌─────────────────────────┐         ┌──────────────────────────────────┐
│ REGION SETTING UNIT      │         │ WEIGHT COMPUTING UNIT            │
│                          │         │                            51    │
│               41         │         │    ┌─────────────────────────┐   │
│                          │         │    │ TRANSMISSION BEAM        │   │
│     ┌──────────┐  42     │         │    │ COMPUTING UNIT           │   │
│     │ MEMORY   │         │         │    └─────────────────────────┘   │
│     └──────────┘         │         │                                  │
│                          │         │                            52    │
│   ┌──────────────────┐   │         │    ┌─────────────────────────┐   │
│   │ WINDOW SIZE      │───┼─────────┼──▶ │ RECEPTION BEAM           │   │
│   │ SETTING UNIT     │   │         │    │ COMPUTING UNIT           │   │
│   └──────────────────┘   │         │    └─────────────────────────┘   │
└─────────────────────────┘         │                            53    │
                                     │    ┌─────────────────────────┐   │
                                     │    │ COMBINING UNIT           │   │
                                     │    └─────────────────────────┘   │
                                     │                            54    │
                                     │    ┌─────────────────────────┐   │
                                     │    │ WEIGHT CALCULATING UNIT  │   │
                                     │    └─────────────────────────┘   │
                                     └──────────────────────────────────┘
                                                                   173
                                     ┌──────────────────────────────────┐
                                     │ SPECTRUM EXTRACTING UNIT          │
                                     └──────────────────────────────────┘
```

# Fig.5

**15**

RECEPTION BEAMFORMER

**17**

**173**

SPECTRUM ANALYZING UNIT

**172**

WEIGHT COMPUTING UNIT

**55**

WEIGHT MULTIPLYING UNIT

**56**

FOURIER-TRANSFORM PERFORMING UNIT

**57**

SPECTRUM TRANSFORMING UNIT

**18**

BIOLOGICAL PHYSICAL QUANTITY MAPPING UNIT

Fig.6

WINDOW REGION 32

Fig.7

COMPUTATION REGION 31

WINDOW REGION 32

SUBREGION 31-i

Fig.8

WINDOW REGION

Fig.9

SPECTRUM ENERGY RATE

WEIGHT BASED ON WAVE PROPAGATION

WEIGHT OF THE RELATED ART

POSITION

RECEIVED SIGNAL FROM TARGET REGION 30

# Fig.10

PHYSICAL QUANTITY COMPUTING PIXEL

# Fig.11

PHYSICAL QUANTITY COMPUTING PIXEL

LARGE

PHYSICAL QUANTITY

SMALL

Fig.12

```
              ┌─────────────┐
              │    START    │
              └──────┬──────┘
                     │
                     ▼                              S11
    ┌────────────────────────────────────────┐
    │              SET REGION                 │
    └──────────────────┬─────────────────────┘
                       │
                       ▼                            S12
    ┌────────────────────────────────────────┐
    │   COMPUTE TRANSMISSION-SIDE SOUND FIELD │
    └──────────────────┬─────────────────────┘
                       │
                       ▼                            S13
    ┌────────────────────────────────────────┐
    │    COMPUTE RECEPTION-SIDE SOUND FIELD   │
    └──────────────────┬─────────────────────┘
                       │
                       ▼                            S14
    ┌────────────────────────────────────────┐
    │      COMPUTE STRENGTH DISTRIBUTION      │
    └──────────────────┬─────────────────────┘
                       │
                       ▼                            S15
    ┌────────────────────────────────────────┐
    │             COMPUTE WEIGHT              │
    └──────────────────┬─────────────────────┘
                       │
                       ▼
              ┌─────────────┐
              │     END     │
              └─────────────┘
```

Fig.13

```
        ┌─────────────┐
        │    START    │
        └──────┬──────┘
               │
               ▼
     ┌───────────────────────┐
     │    MULTIPLY WEIGHT     │  ⟋ S22
     └───────────┬───────────┘
                 │
                 ▼
     ┌───────────────────────────┐
     │ PERFORM FOURIER TRANSFORM  │  ⟋ S23
     └───────────┬───────────────┘
                 │
                 ▼
     ┌───────────────────────┐
     │    EXTRACT SPECTRUM    │  ⟋ S24
     └───────────┬───────────┘
                 │
                 ▼
        ┌─────────────┐
        │     END     │
        └─────────────┘
```

## INTERNATIONAL SEARCH REPORT

| International application No. |
|---|
| PCT/JP2016/069905 |

A. CLASSIFICATION OF SUBJECT MATTER
*A61B8/14*(2006.01)i

According to International Patent Classification (IPC) or to both national classification and IPC

B. FIELDS SEARCHED

Minimum documentation searched (classification system followed by classification symbols)
A61B8/14

Documentation searched other than minimum documentation to the extent that such documents are included in the fields searched
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 3202969 B2 (Fujitsu Ltd.), 27 August 2001 (27.08.2001), entire text; all drawings (Family: none) | 1–12 |
| A | JP 2014-61265 A (Canon Inc.), 10 April 2014 (10.04.2014), entire text; all drawings & US 2014/0064022 A1 | 1–12 |
| A | JP 2015-213575 A (Toshiba Corp.), 03 December 2015 (03.12.2015), entire text; all drawings & US 2015/0320396 A1 | 1–12 |

☐ Further documents are listed in the continuation of Box C. ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 09 September 2016 (09.09.16) | 20 September 2016 (20.09.16) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2001170046 A